# EUROPEAN PATENT APPLICATION

(11) **EP 4 537 755 A1**
(43) Date of publication of application: **16.04.2025**
(21) Application number: 23383036.3
(22) Date of filing: 09.10.2023
(51) Int. Cl.: A61B 5/291

(54) **BIOSIGNAL REGISTERING SYSTEM**

(71) Applicant: Time is Brain, SL, 08916 Badalona (Barcelona) (ES)
(72) Inventor: MARTÍNEZ PIÑEIRO, Alicia, 08020 Barcelona (ES); RUIZ VEGA, Gisela, 08020 Barcelona (ES); COLL CANTÍ, Jaume, 08020 Barcelona (ES); IRIONDO LOZANO, Maite, 08290 Barcelona (ES); ROVIRA COSTA, Enric, 08290 Barcelona (ES)
(74) Representative: Hoffmann Eitle

(57) **Abstract**

A registering system comprising at least one electrode, and at least one dispensing unit. The dispensing unit is configured to be placed over the at least one electrode and provide a predefined amount of electrolytic gel between the at least one electrode and the skin surface of the user. The registering system is characterised in that the predefined amount of electrolytic gel is of at least 1.75 ml per cm² of surface of electrode in contact with the skin through said electrolytic gel, more preferably at least 2.3 ml per cm² of surface of electrode in contact with the skin through said electrolytic gel.

## Description

### Technical field of the invention

The present invention belongs to the field of medicine. Particularly, the present invention relates to the field of signal acquisition, more particularly to a registering system including a electroencephalography registering system and uses thereof.

### Background of the invention

Electroencephalography (EEG) is a well-known technique to register brain activity. The biosignals detected by EEG represent the postsynaptic potentials of pyramidal neurons in the neocortex and allocortex of the brain. It is mainly practiced on the scalp (scalp EEG) and its main advantage compared to other electrogram registering techniques, such as electrocorticography (ECoG) and variations of EEG such as subdural EEG, intracranial EEG, or stereotactic EEG is the non-invasiveness and simplicity of the procedure. However, this comes with a cost, mainly a low signal-to-noise ratio (SNR). As the signal is weak and has to travel through different structures (pia mater, arachnoid, dura mater, bone and skin, among others), which are not relatively fixed between each other, the signal is exposed to noise that results in such a low SNR.

In order to compensate for such a low SNR different solutions have been proposed, including amplifying units and signal processing techniques. For applications where the precision and quality of the signal is essential, invasive solutions, such as ECoG are preferred. This also solves another limitation of EEG, the low spatial resolution. Given the distance between the source and the registering point, the source location is not easy to determine, considering the small size of the pyramidal neurons and the complexity of the brain.

However, there are situations wherein the spatial resolution is not a problem, while obtaining EEG data in the least amount of time is essential, or wherein an intervention is not recommended or desirable. This is for example the case of emergency rooms, wherein EEG can be used to obtain useful data which the doctors can use to take decisions, or in ICU, wherein the EEG can also provide insights on the status of the patient and evaluate the progression of the same. In these scenarios, there is still a need to make the signal as clean as possible (with the highest SNR) in the shortest amount of time.

EEG involves providing one or more electrodes on the patient's scalp skin, to register the activity. The state-of-the-art solutions involve the use of helmets, caps or specifically designed head mounts, wherein the EEG electrodes are located or predetermined to be located on certain locations. This is because the EEG signals are registered over the scalp surface, and each scalp location represents the location in the brain cortex immediately below. Therefore, following certain standards such as the International 10-20 system, helps to easily locate the electrode above the area of interest of the brain cortex.

The interface between the electrode and the scalp skin is the main source of noise. This is because the electrode is hardly perfectly stable over the scalp surface (considering hair and debris), and because the electrode surface and the scalp surface are not perfectly parallel (irregularities, skin folds, natural curves, skull crests) and therefore the contact are between both is not stable.

To reduce the signal noise and the impedance of the scalp-electrode interface, electrolytic solutions are preferably provided in this interface. It is also common to prepare the scalp surface prior to the EEG register, for example by cleaning the debris to expose clean skin. However, these procedures are time consuming and they are usually either performed in a rush, leading to measurements which are not optimal, or they delay the EEG sample acquisition.

As in EEG recording systems, many other registering systems for biosignals also require complex and time-consuming protocols in order to obtain a signal with enough quality, which in some scenarios are a burden for the medical specialists. Therefore, the above difficulties can be also extrapolated to other registering systems and modalities through superficial electrodes.

There is therefore a need for a registering system that can provide signals with the highest SNR possible in a fast way, and preferably reducing any user variability, such that the results can be obtained in under any circumstances. In particular, there is a need for such a system for registering EEG given that the quality of the signal is essential for a proper assessment of the same.

### Summary of the invention

A first aspect of the invention is directed to a registering system comprising at least one electrode, and at least one dispensing unit. The dispensing unit is configured to be placed over the at least one electrode and provide a predefined amount of electrolytic gel between the at least one electrode and the skin surface of the user. The registering system is characterised in that the predefined amount of electrolytic gel is of at least 1.75 ml per cm² of surface of electrode in contact with the skin through said electrolytic gel, more preferably at least 2.3 ml per cm² of surface of electrode in contact with the skin through said electrolytic gel.

In a preferred embodiment of the first aspect of the invention, the registering system is an electroencephalography registering system, further comprising a head mount configured to attach to the head of a user. In this preferred embodiment, the at least one electrode is configured to be placed within the head mount in a predefined location over the scalp of the user, and the predefined amount of electrolytic gel is provided between the at least one electrode and the scalp surface.

In another preferred embodiment, the dispensing unit is configured to provide the predefined amount of electrolytic gel when a force is applied to a certain region of the dispensing unit. More preferably, the dispensing unit comprises a plunger or a flexible surface and the predefined amount of electrolytic gel is provided when the force is applied to the plunger or the flexible surface, respectively. Even more preferably, the dispensing unit is configured to provide the predefined amount of electrolytic gel when a rotational force is applied to the plunger.

In an alternative preferred embodiment, the dispensing unit comprises a seal and is configured to provide the predefined volume by gravity once the seal has been defunctionalized. More preferably, the registering system further comprises a seal breaking device, configured to be attached to the dispensing unit and to break the seal by the attachment mechanism to the dispensing unit.

In a preferred embodiment according to any one of the previous embodiments, the at least one electrode comprises an opening and wherein the dispensing unit is configured to provide the predefined amount of electrolytic gel though the opening.

In another preferred embodiment according to any one of the previous embodiments, the electrolytic gel comprises an abrasive component, and wherein the dispensing unit is further configured to prepare and clean the skin of the user through the provision of the electrolytic gel comprising the abrasive component. More preferably, the abrasive component comprises pumice.

In another preferred embodiment according to any one of the previous embodiments, the at least one electrode comprises any of the materials selected from the following list: gold, steel, silver, silver - silver chloride and sintered silver-silver chloride.

In another preferred embodiment according to any one of the previous embodiments, the system comprises more than one registering electrode and at least one ground electrode. More preferably, when the registering system is an electroencephalography registering system, the registering electrodes are located in evoked potential locations of the scalp. Even more preferably the registering electrodes are located in sensory evoked potential positions, even further preferably in the Cz', C3' and C4' respective locations of the head mount according to the international 10-20 system. In this more preferred embodiment, the ground electrode is located in a mastoid location of the head mount.

A second aspect of the invention is directed to the use of the electroencephalography registering system according to any one of the embodiments of the first aspect of the invention when the registering system is an electroencephalography registering system to register an EEG signal. More preferably, to register an evoked potential (EP), even more preferably an somatosensory evoked potential (SEP).

In a preferred embodiment of the second aspect of the invention, the EEG signal to be registered is the N20 SEP.

### Brief description of the drawings

To enable a better understanding of the present disclosure, and to show how the present disclosure may be carried out, reference will now be made, by way of example only, to the accompanying schematic drawings, wherein:
Figure 1 shows a schematic view of a registering system according to one or more embodiments of the invention.
Figure 2 shows another schematic view of an electrode and a dispensing unit according to one or more embodiments of the invention.
Figure 3 shows (a) a perspective view, (b) a cut-through view before the provision of the electrolytic gel and (c) a cut-through view after the provision of the electrolytic gel of a dispensing unit according to one or more embodiments of the invention.
Figure 4 shows (a) a cut-through view before the provision of the electrolytic gel, (b) a perspective view, and (c) a cut-through view after the provision of the electrolytic gel of another dispensing unit according to one or more embodiments of the invention.
Figure 5 shows a perspective view of another dispensing unit (a) before the provision of the electrolytic gel, (b) after configuring it for a new provision of the electrolytic gel and (c) after the provision of the electrolytic gel.
Figure 6 shows another schematic view of an electrode and a dispensing unit according to one or more embodiments of the invention.
Figure 7 shows two graphs for the registered signal using with two different electrolytic gels comprising an abrasive component according to one or more embodiments of the invention.
Figure 8 shows two graphs for the registered signal using a registering system two different electrolytic gels according to one or more embodiments of the invention.
Figure 9 shows a graph for (a) a set of measurements and (b) the detrended signal obtained by a registering system according to one or more embodiments of the invention.
Figure 10 shows another graph for (a) a set of measurements and (b) the detrended signal obtained by a registering system according to one or more embodiments of the invention.
Figure 11 shows another graph for (a) a set of measurements and (b) the detrended signal obtained by a registering system according to one or more embodiments of the invention.
Figure 12 shows (a) a bottom view and (b) a lateral view of a dispensing unit with a provision tube according to one or more embodiments of the invention.

### Description of the invention

### Definitions

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

It is noted that the term "about", as used herein, refers to +/- 30%, preferably +/- 20%, preferably +/- 15%, more preferably +/- 10%, of the indicated referred value.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of does not exclude materials or steps that do not materially affect the basic and novel characteristics of the claim.

The term "dispensing unit" in the context of the present invention preferably refers to a container configured to provide or dispense its content when certain conditions are met. Preferably it comprises means to provide its content, such as the electrolytic gel, and the conditions may be the actuation of any element of the same, such as a plunger.

The term "electrolytic gel" in the context of the present invention refers to an electrically conductive fluid, preferably a gel, which may comprise electrolytes configured to increase its electrical conductivity and/or abrasive substances.

### Description

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

Although the present invention and its figures may be exemplified through an EEG system, it is understood that the invention herein provided can be extrapolated to any kind of registering systems as long as it is suitable for registering biosignals non-invasively. Therefore, it shall not be understood the EEG as a limiting factor in order to apply this invention.

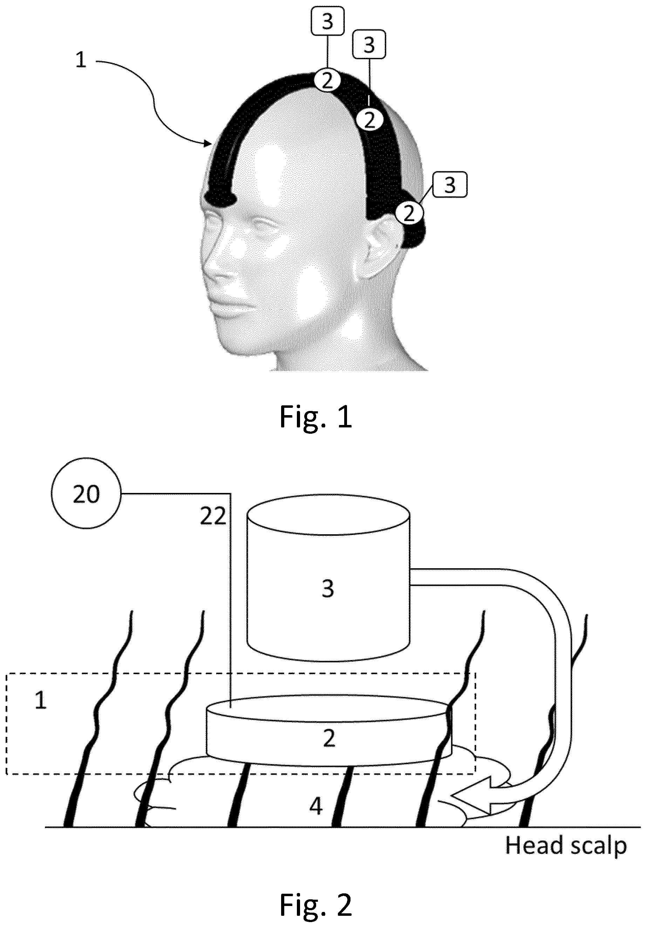

A first aspect of the invention relates to a registering system. As shown schematically with respect to Fig. 1 it comprises at least one electrode 2, and at least one dispensing unit 3 configured to be placed over the at least one electrode 2.

Fig. 2 shows a diagram of a registering system according to the present invention. The registering system, comprises at least one electrode 2. The at least one electrode 2, may be any electrode suitable for registering biosignals, as known in the art. Thus, the al least one electrode 2, may take different shapes and sizes. For example, the electrode may be bigger, thinner or may comprise more than one materials. It is noted than when the system comprises more than one electrode 2, the electrodes 2 may be different between them.

The registering system also comprises at least one dispensing unit 3. The dispensing unit 3 is configured to be placed over the at least one electrode 2 and provide a predefined amount of electrolytic gel 4 between the at least one electrode 2 and the skin surface of the user. The dispensing unit may be therefore any unit suitable for containing an electrolytic gel 4 and dispose it when certain conditions are met. These conditions may be the actuation of the dispensing unit 3, but it also may be also a certain position of the dispensing unit 3, such as the placement of the at least one dispensing unit 3 over the at least one electrode 2.

The at least one dispensing unit 3 may be configured to be placed over the at least one electrode 2, in different ways. For example, the at least one dispensing unit 3 may be integrated over the at least one electrode 2. Alternatively, it may be removably attached, for example through bolts, a snap connection, a thread or any well-known connection. This may allow for example removing the at least one dispensing unit 3 once the electrolytic gel 4 has already been provided between the at least one electrode (2) and the skin surface of the user.

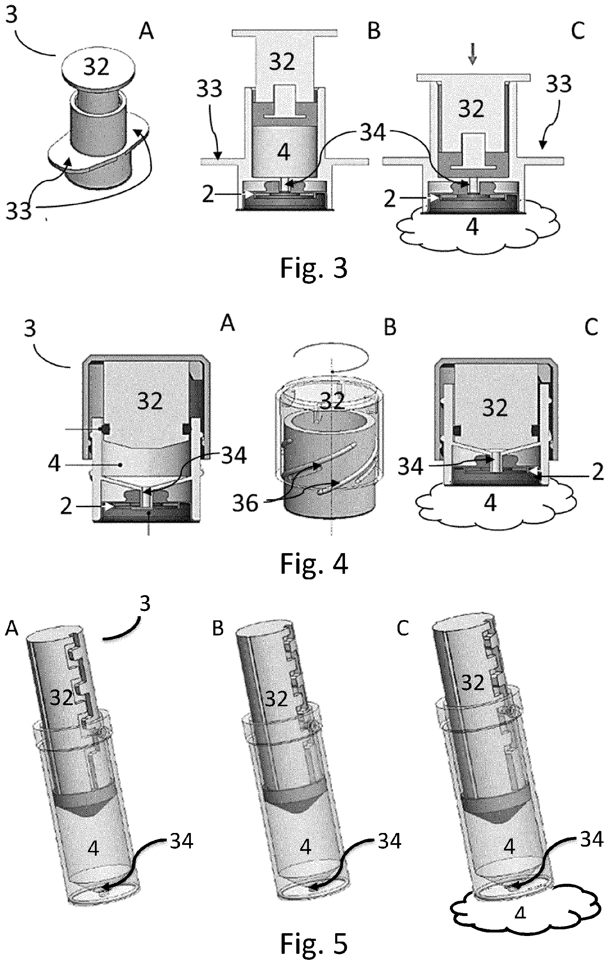

The at least one dispensing unit 3 is configured to be provide a predefined amount. It may be a predefined amount because the whole content of electrolytic gel 4 within the dispensing unit 3 is the one to be provided, and therefore when certain conditions are met the whole content of the dispensing unit 3 is provided. Alternatively, it can be that the dispensing unit 3 is configured to provide a portion of its content in each occasion certain conditions are met, as will be later explained with reference to Fig. 5.

It is noted the dispensing unit 3 can be configured to provide the predefined amount of electrolytic gel 4 between the at least one electrode 2 and the skin surface of the user in different ways. For example, the dispensing unit may comprise one or more means to conduct the provision of the electrolytic gel 3, and the means may be configured to direct such provision to the area between the at least one electrode 2 and the skin surface of the user.

It is also noted that the dispensing unit 3 of the diagram of Fig. 2 has a cylindrical shape. However, it may be noted that the dispensing unit 3 may take other shapes and sizes, adapted to different preferences. For example, it may be desirable to have a dispensing unit wider or thinner, or taller or shorter depending on the use of the registering system. Moreover, it is noted than when the system comprises more than one dispensing units 3, the dispensing units 3 may be different between them.

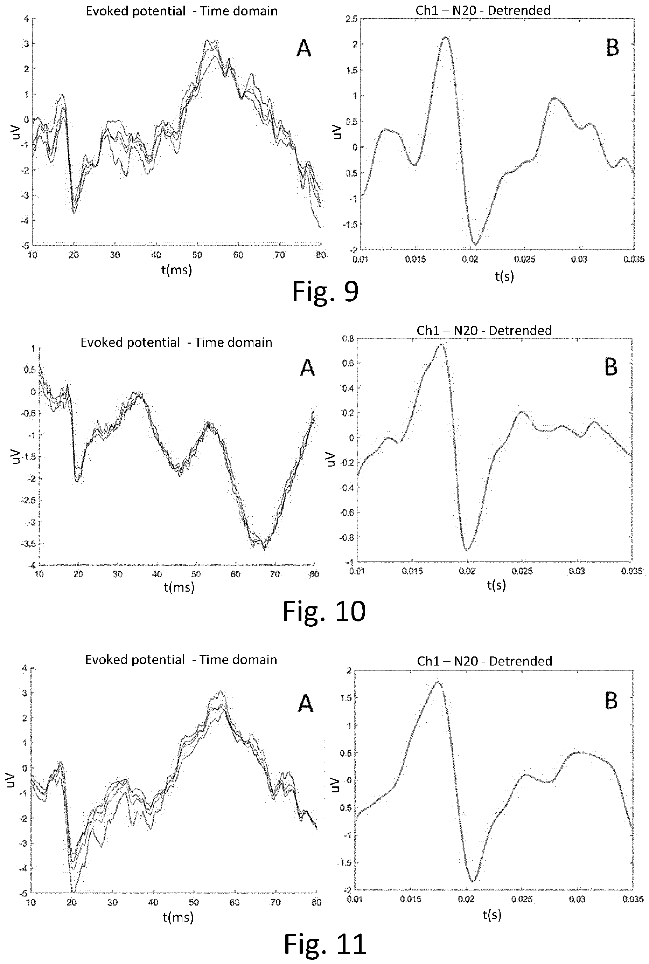

According to a preferred embodiment, the registering system is characterised in that the predefined amount of electrolytic gel 4 is at least 1.75 ml per cm² of surface of electrode in contact with the skin through said electrolytic gel. As will be later explained in Example 2, and in reference to Figs. 9 and 11, such amount of electrolytic gel 3 and preferably in combination with the provision of an electrolytic gel 3 ensures an electrode-skin impedance lower than 10k ohms, which in turn ensures that the SNR is increased.

In a preferred embodiment, the predefined amount of electrolytic gel 4 is at least 1.8 ml per cm², more preferably at least 1.9 ml per cm² even more preferably at least 2 ml per cm², 2.1 ml per cm² or 2.2 ml per cm². In a further very preferred embodiment, the predefined amount of electrolytic gel 4 is at least 2.3 ml per cm² of surface of electrode in contact with the skin through said electrolytic gel. Advantageously, having a predefined amount of electrolytic gel 4 of at least 2.3 ml per cm² makes the electrode-skin impedance to be optimal as shown also in Example 2.

In another preferred embodiment, the predefined amount of electrolytic gel 4 is at most 6 ml per cm² of surface of electrode in contact with the skin through said electrolytic gel. Advantageously, this limitation further prevents having a dispensing unit 3 too big and avoids the provision of an excess of electrolytic gel 4 which could be counterproductive of two close electrodes 2 come in contact through the same electrolytic gel 4.

Advantageously, the registering system herein described can provide signals with the highest SNR possible in a fast way, as the same system comprises the required electrolytic medium, and because by its amount and type of electrolytic medium, it ensures the skin impedance is lower than 10 kΩ.

It is noted that Fig. 1 comprises many features that are not essential and may be omitted or may be different in other embodiments of the present invention. For example, the registering system may not comprise a mount 1. And if it comprises a mount 1, it may not be a head mount but any other type of mount configured to mount the electrodes to a certain region of the body of the user. It is also noted that in Fig. 1 there are three electrodes 2 and three dispensing units 3, although in some other embodiments they may be one, two or more than three elements of any of the electrodes 2 and/or dispensing unit 3. Also, the at least one electrode 2 and the at least one dispensing unit 3 are schematically represented, but it can take any shape or size, and can be located anywhere. It is therefore understood that in other embodiments of the present invention, these features may be different to those as depicted in Fig. 1.

Moreover, it is noted that Fig. 2 also comprises many features that are not essential and may be omitted or may be different in other embodiments of the present invention. For example, the at least one electrode 2 may not be placed over the head scalp, but over any other skin surface of the user. It is also noted that the scalp surface comprises many hairs, that are however just figurative and in some other embodiments the skin surface may not comprise hairs at all. It is also noted that the size of the hairs are not proportionate to the registering system, and shall not be considered to limit the size of any of the components of the registering system. Also, it is noted that the at least one electrode 2 and the at least one dispensing unit 3 are represented in the form of cylinders, however, in other embodiments, the at least one electrode 2 and the at least one dispensing unit 3 may have other shapes or size. It is also noted that the size proportion between the at least one electrode 2 and the at least one dispensing unit 3 may be different. For example, the at least one electrode 2 may be significantly thinner than the at least one dispensing unit 3. It is also noted that the at least one dispensing unit 3 is depicted providing the electrolytic gel 4 in a symbolic way laterally, although it may be configured to provide it in other ways, as will be later described in reference to other embodiments. Also, the registering system according to Fig 2 comprises processing means 20 and connection means 22, that in other embodiments may not be comprised within the registering system. Finally, the registering system may not comprise a mount 1.

According to a preferred embodiment of the first aspect of the invention and as shown in Fig 1, the registering system is an electroencephalography (EEG) registering system. The EEG registering system further comprises a head mount 1 configured to attach to the head of a user, wherein the at least one electrode 2 is configured to be placed within the head mount 1 in a predefined location over the scalp of the user. The predefined amount of electrolytic gel 4 is therefore provided between the at least one electrode 2 and the scalp surface.

The head mount can take several shapes, such as in the form of a helmet, a band, a cap, a bandana or a head-scurf, among others, all of them configured to be placed over the head skin of a subject. The skilled person may note that there al multiple variations and alternative in which a head mount 1 may be defined, all of them comprised within this embodiment. Moreover, the head mount 1 may be configured to be placed over the head skin of a subject by different means, such as by having a certain elastic element, like an elastic band, or attaching means like a Velcro-alike closure, or just be designed with a shape configured to attack to the head, either passively, like a hat or actively, like a wearable VR glasses with selfadjustment bands. Thus, the present embodiment is not limited by any means to a certain particular type of mount 1 as long as it comprises the following features that will be now described.

The at least one electrode 2 is therefore configured to be attached to be placed within the head mount. The at least one electrode 2 may be therefore embedded into the head mount 1, or the head mount may comprise specific locations configured to place the at least one electrode 2 therein. The at least one electrode 2 may be therefore, attached to the head mount 1 by different means, for example through a Velcro system, a snap button or a specific pocket in the head mount. It is also noted that the electrode may be thus connected to some processing means 20 through a connection means 22, and in such embodiments, the head mount may comprise specifically pathways configured to held or contain such connection means 22. In some embodiments the head mount may also integrate such connection means an even the processing means 20. The at least one electrode 2 and the at least one dispensing unit 3 may take different shapes and sizes as above explained.

The at least one electrode 2 is configured to be placed within the head mount 1 in a predefined location over the scalp of the user. because the head mount is configured to hold the electrode in certain location which is desired. This can vary depending on the application. Therefore, the head mount 1 can comprise many locations, for example following the international 10-20 system, and the at least one electrode only be located in one of such locations. Alternatively, the at least one electrode 2 may be integrated into the head mount 1 or the head mount comprise just the required locations where to locate the at least one electrode 2.

Advantageously, the EEG registering system herein described can provide signals with the highest SNR possible in a fast way, as the same system comprises the required electrolytic medium, and because by its amount and type of electrolytic medium, it ensures the skin impedance is lower than 10 k ohms. Thus, a user simply places the head mount onto the head of the patient and by interacting with the at least one dispensing unit 3, the skin impedance is significantly reduced to the desired levels. This means the EEG registering system herein described effectively reduces any user variability, such that the results can be obtained in under any circumstances, as the electrolytic medium is provided by the dispensing unit. Moreover, it further helps to avoid the instability provided by the presence of hairs between the scalp surface and the electrode in some of the patients.

It is noted the head unit 1 of Fig. 1 may take many other different shapes, and the at least one electrode and at least one dispensing unit 3 may be located in other locations.

According to a particular preferred embodiment, as shown with respect to any of Figs. 3 to 5 the dispensing unit 3 is configured to provide the predefined amount of electrolytic gel 4 when a force is applied to a certain region of the dispensing unit 3.

In this particular preferred embodiment, the dispensing unit 3 preferably has a certain region to apply a force in order to actuate it and provide the predefined amount of electrolytic gel 4. For example, the dispensing unit 3 may compromise a flexible membrane which can be squeezed or pressed, in order to increment the pressure within its internal space wherein the electrolytic gel 4 is comprised, Therefore, the electrolytic gel 4 is forced to be provided between the at least one electrode 2 and the skin surface of the user, preferably through an opening 34. Alternatively, a mechanically or electromechanically system, such as a plunger or an electromechanical mechanism may be provided within the dispensing unit 3 to make the electrolytic gel 4 to be provided between the at least one electrode 2 and the skin surface of the user, preferably through an opening 34. Advantageously, this allows for a simple to meet conditions for the at least one dispensing unit 3 to provide the predefined amount of electrolytic gel 4.

According to a more preferred embodiment, and as shown in Figs, 3 to 5, the dispensing unit 3 comprises a plunger 32 or a flexible surface and the predefined amount of electrolytic gel 4 is provided when the force is applied to the plunger 32 or the flexible surface, respectively.

For example, according to a particular embodiment and as shown in Fig. 3A the dispensing unit 3 may comprise a plunger 32. In Fig. 3B it is shown that before the provision of a force onto the plunger, the electrolytic gel 4 is comprised within the dispensing unit 3. After the force has been applied, and as shown in Fig. 3C, once the plunger 32 has been pressed, the electrolytic gel 4 has been provided between the electrode 2 and the skin of the user (not shown).

It is noted that Fig 3 comprises many features that are not essential and may be omitted or may be different in other embodiments of the present invention. For example, it can be observed that the electrode 2 is significantly thinner than the dispensing unit 3, and that the dispensing unit is placed over the electrode 2 such that the electrode 2 is practically embedded within the dispensing unit 3. However, this may not be the case in other embodiments of this more preferred embodiment. The dispensing unit 3 and the plunger 32 may take many other different shapes. Also the dispensing unit of Figs. 3A-3C comprises a particular opening 34 through which the electrolytic gel 4 is provided. However, in the embodiments the opening 34 can take other shape, be located in other place or the dispensing unit 3 may comprise more than one opening 34. Finally, it is noted that the dispensing unit 3 comprises a pair of ears 33 sticking out that help in the actuation of the plunger 32. In other embodiments these structures 33 may take other shape, may be more than two or may not be present at all in the dispensing unit 3.

According to a more particular embodiment, and as shown with respect to fig 4, the dispensing unit 3 is configured to provide the predefined amount of electrolytic gel 4 when a rotational force is applied to the plunger 32. For example, the dispensing unit 3 may comprise one or more rotational means 36, such as that the rotation of the plunger 32 with respect to the dispensing unit 3, forces the axial displacement of the plunger 32, therefore squeezing the content of the disposing unit and providing the predefined amount of electrolytic gel 4 between the at least one electrode 2 and the skin surface of the user.

For example, according to a particular embodiment and as shown in Fig. 4A the electrolytic gel 4 is comprised within the dispensing unit 3. In Fig 4B it can be seen that the dispensing unit 3 further comprises rotational means 36 such that the rotation of the plunger 32 with respect to the dispensing unit 3, forces the axial displacement of the plunger 32. Finally, after the rotational force has been applied, and as shown in Fig. 4C, the electrolytic gel 4 has been provided between the electrode 2 and the skin of the user (not shown).

It is noted that Fig. 4 comprises many features that are not essential and may be omitted or may be different in other embodiments of the present invention. For example, the dispensing unit 3 and the plunger 32 may take many other different shapes. Also it is noted that the rotational means 36 of Fig. 4 have the form of rails, but in other embodiments, the rotational means may be located in the plunger itself, or may be internally comprised within the dispensing unit 3, and may further take other solutions to make the plunger axially displace once the plunger 32 is rotated. Also the dispensing unit of Figs. 3A-3C comprises a particular opening 34 through which the electrolytic gel 4 is provided. However, in the embodiments the opening 34 can take other shape, be located in other place or the dispensing unit 3 may comprise more than one opening 34.

In Fig. 5 is shown an alternative embodiment according to one or more embodiments of the present invention. It is noted that the at least one dispensing unit 3 may be configured to provide more than one time the predefined amount of electrolytic gel 4 between the at least one electrode 2 and the skin surface of the user (not shown).

For example, in Fig. 5A, it can be seen that the electrolytic gel 4 is comprised within the dispensing unit 3. In Fig 5B it can be seen that the plunger 32 of the dispensing unit 3 has been rotated, which further enables the provision of the predefined amount of electrolytic gel 4 once. This is because the plunger 32 and the dispensing unit 3 comprise a mechanism that limits the axial translation of the plunger 32 with respect to the dispensing unit 3. Finally, after the axial force has been applied, and as shown in Fig. 5C, the electrolytic gel 4 has been provided between the electrode 2 and the skin of the user (not shown). Advantageously, the dispensing unit 3 may be further used to provide the predefined amount of electrolytic gel 4 to other electrodes 2, as it still comprises electrolytic gel 4.

It is noted that Fig. 5 comprises many features that are not essential and may be omitted or may be different in other embodiments of the present invention. For example, the dispensing unit may have other shape, size, and may comprise any other mechanism to ensure that the predefined amount of electrolytic gel 4 is provided while containing a greater amount of electrolytic gel within itself. For example, it may comprise an actuated motor that in every actuation of the same it only displaces the plunger 32 axially with respect to the dispensing unit 3 a determined length, which ensures the predetermined amount of electrolytic fluid 4 is provided. This may be for example controlled through a processing unit such as a microcontroller. Also the dispensing unit of Figs. 5A-5C comprises a particular opening 34 through which the electrolytic gel 4 is provided. However, in the embodiments the opening can take other shape, be located in other place or the dispensing unit 3 may comprise more than one opening 34.

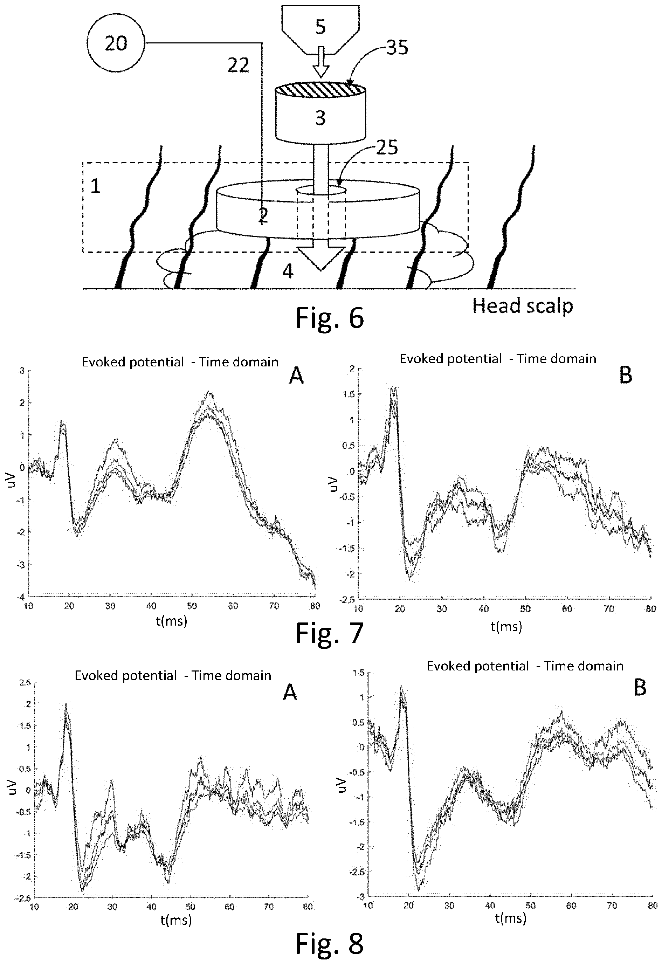

According to a preferred alternative embodiment, and as shown for example in Fig. 6 the dispensing unit 3 comprises a seal 35 and is configured to provide the predefined volume by gravity once the seal 35 has been defunctionalized. The term "defunctionalized" in the context of the present invention may be understood as disabling or making the seal 35 no longer functional by any procedure. The seal 35 may be defunctionalized, for example, by either removing the seal 35 from the dispensing unit 3, or by perforating or breaking the seal 35, such that the sea 35l no longer holds a vacuum. Therefore, the seal 35 creates a vacuum within the dispensing unit 3, which avoids the electrolytic gel 4 to be provided between the at least one electrode 2 and the skin surface of the user, preferably through an opening 34. Once the seal 35 is defunctionalized, air can enter the dispensing unit 3 and the electrolytic gel 4 can flow outwards towards the space between the electrode 2 and the skin of the user. It is noted that this design requires the orientation of the registering system, particularly the dispensing unit 3, such that the dispensing unit 3 is positioned such that the means wherein the electrolytic gel 4 are provided are located closer to the ground than the current location of at least part of the electrolytic gel. This is, the means, such as the opening 34, shall be located in the lower part of the dispensing unit 3 once located in place over the electrode 2 over the skin of the user. This ensures that once the seal 35 is defunctionalized, the gravity effectively biases the electrolytic gel towards this provision means. For example, as shown with respect to Fig. 6 the dispensing means may be provided on the lower post part if the dispensing unit 3, and the seal 35 on its uppermost part. The disposing means may be an orifice 34. Alternatively, the seal 35 may be located covering the dispensing means, such that when defunctionalized, the electrolytic gel 4 can exit through such means, such as the opening 34.

It is noted that Fig. 6 comprises many features that are not essential and may be omitted or may be different in other embodiments of the present invention. For example, the at least one electrode 2 may not be placed over the head scalp, but over any other skin surface of the user. It is also noted that the scalp surface comprises many hairs, that are however just figurative and in some other embodiments the skin surface may not comprise hairs at all. It is also noted that the size of the hairs are of Fig. 6 are not proportionate to the registering system, and shall not be considered to limit the size of any of the components of the registering system. Also, it is noted that the at least one electrode 2 and the at least one dispensing unit 3 are represented in the form of cylinders, however, in other embodiments, the at least one electrode 2 and the at least one dispensing unit 3 may have other shapes or size. It is also noted that the size proportion between the at least one electrode 2 and the at least one dispensing unit 3 may be different. It is also noted that the at least one dispensing unit 3 is depicted providing the electrolytic gel 4 through an opening of the electrode 2, although it may be configured to provide it in other ways, such as laterally. Also, the registering system according to Fig 2 comprises processing means 20, connection means 22 and a seal breaking device 5, that in other embodiments may not be comprised within the registering system. Finally, the registering system may not comprise a mount 1.

Advantageously, this embodiment does not necessarily require applying any force over the dispensing unit 3 when placed over the electrode 2, as long as the seal 35 is defunctionalized. This might be done before having the dispensing unit 3 placed over the electrode 2, which could be of benefit if the skin of the patient is sensitive, or if for any reason it is not desirable to apply a force over the surface of the skin.

According to a more particular embodiment, and as shown in Fig. 6, the registering system further comprises a seal breaking device 5, configured to be attached to the dispensing unit 3 and to break the seal 35 by the attachment mechanism to the dispensing unit 3. Advantageously, the seal breaking device 5 may be configured to break the seal 35 with a simple movement. Also when the registering system comprises more than one dispensing unit 3 a single seal breaking device 5 can break the seals 35 of more than one dispensing unit 3. It is noted that the shape of the seal breaking device 5 is just illustrative, and that in other embodiments of this more particular embodiment it can take other shapes. For example, it may comprise particular attachment mechanism and means configured to ensure the seal 35 is defunctionalized by the attachment to the dispensing unit 3.

According to another preferred embodiment, as shown also with reference to Fig. 6 the at least one electrode 2 comprises an opening 25 and the dispensing unit 3 is configured to provide the predefined amount of electrolytic gel though the opening 25. Advantageously, this facilitates the provision of the electrolytic gel 4 between the electrode 2 and the skin of the user, as the opening 25 provides a direct access for the electrolytic gel 4 to the space between the electrode 2 and the skin of the user. It is noted that the opening 25 might not be centred in the electrode 2, and may be therefore biased towards one side. It is also noted that the opening may take several different shapes, and may be therefore not be cylindrical. It may therefore take an elliptic shape, a ring shape or may change its width as it get closer to the skin. The electrode 2 may also comprise more than one opening 25 configured to provide the predefined amount of electrolytic gel 4.

When the registering system comprises more than one electrode 2, in some embodiments the opening 25 may be present in only one of the electrodes 2, on some of the electrodes 2 or in all the electrodes 2. When there is more than one electrode 2 with an opening 25, it may be different between electrodes in terms of shape size and/or location within the electrode 2, and the number of openings 25 in each electrode.

According to a preferred embodiment, the opening 25 is placed in the centre of the electrode. It is noted that for a certain electrode shape, the centre may be defined by any of its circumcentre, centroid or incentre which may be easily computed by the skilled person. Advantageously, this allows the provision of the electrolytic gel 4 between the at least one electrode 2 and the skin surface of the user in a simple and homogeneous way.

According to another preferred embodiment, the electrolytic gel 4 comprises an abrasive component. Thus, the dispensing unit 3 is further configured to prepare and clean the skin of the user through the provision of the electrolytic gel 4 comprising the abrasive component.

Advantageously, this allows for the registering system To be configured to not only provide the predefined amount of electrolytic gel 4 between the at least one electrode 2 and the skin surface of the user, but to also prepare and clean the skin of the user at the same time. As the abrasive component is comprised within the electrolytic gel 4, the provision of the electrolytic gel 4 by any of the possible ways to actuate the dispensing unit 3 above-described, produces both effects. Therefore, as the skin debris increases the impedance, the registering system according to this embodiment, further enables the provision of signals with the highest SNR possible in a fast way.

As will be explained later on in Example 1 with respect to Figs 7 and 8, it has been shown that when the electrolytic gel 4 comprises an abrasive component, the SNR of the signals registered is increased, as the impedance of the skin surface is reduced. Therefore, having a registering system not providing the electrolytic gel 4 further comprising an abrasive component it further solves the problem of obtaining a system that can easily and quickly provide a stable and reliable signal with high SNR.

In a more preferred embodiment, the abrasive component comprises any of the materials selected from the following list: pumice. Advantageously, pumice has been shown to be abrasive and not reduce significatively the conductivity of the electrolytic gel, so its therefore suitable as an abrasive material for a registering system as defined in the present invention.

According to another preferred embodiment, the at least one electrode 2 comprises any of the materials selected from the following list: gold, steel, silver, silver - silver chloride and sintered silver-silver chloride. Advantageously, these materials have a small impedance, so they further help to reduce the skin barrier impedance, and therefore increase the SNR of the acquired EEG signal.

According to another preferred embodiment, the system comprises more than one registering electrode 2a and at least one ground electrode 2b. Advantageously, this enables registering several signals with the same registering system. Therefore, the ground electrode 2b can serve as ground electrode of all the registering electrodes 2a. It is understood that one or more of the registering electrodes 2a may be further used as a reference electrode.

In a more preferred embodiment wherein the registering system is a EEG system, the registering electrodes are located in evoked potential locations of the scalp, preferably in sensory evoked potential positions more preferably in the Cz', C3' and C4' respective locations of the head mount 1 according to the international 10-20 system, and the ground electrode 2b is located in a mastoid location of the head mount 1. 1. Therefore, the ground electrode may be comprised in the left or light mastoid locations or in both mastoid locations. In a preferred embodiment, the ground electrode 2b is located in the left mastoid location. Advantageously, this EEG registering system is suitable for registering somatosensory evoked potentials (SEPs), more preferably, N20 SEPs.

According to a particular preferred embodiment, the registering system is a EEG system suitable for registering the N20 SEP, and the registering electrodes are located in the Cz', C3' and C4' respective locations of the head mount 1 according to the international 10-20 system, and the ground electrode 2b is located in the mastoid locations of the head mount 1. Therefore, the ground electrode may be comprised in the left or light mastoid locations or in both mastoid locations. In a preferred embodiment, the ground electrode 2b is located in the left mastoid location.

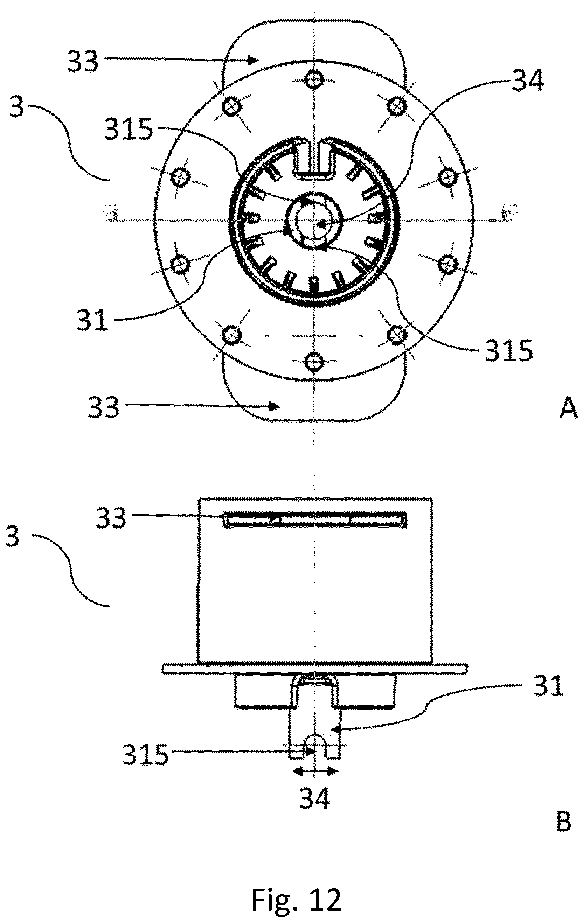

According to a preferred embodiment of any of the previous embodiments and as shown with respect to Fig. 12, the opening 34 is provided through a provision tube 31, the provision tube 31 configured to close the gap between the dispensing unit 3 and the skin of the user. Advantageously, the provision tube 31 facilitates the provision of the predefined amount of electrolytic gel 4 between the at least one electrode 2 and the skin surface of the user.

In a more preferred embodiment, the provision tube 31 comprises a flexural modulus between 1100 and 1300MPa. Preferably the provision tube 31 comprises a flexural modulus between 1100 and 1200MPa, even more preferably a flexural modulus of essentially 1140 MPa. Advantageously, a provision tube 31 with a flexural modulus within these values provides a material firm enough to maintain its shape when provided onto the user's skin, but with enough flexibility to be gentle with the user's skin. This of much relevance in clinical applications, wherein the collaboration of the patient with the registration is essential to ensure an adequate recoding of the biosignals. If the user (patient) is not comfortable with the dispensing unit 1 because the material of the provision tube 31 in contact with the skin is too stiff, the user may sense a discomfort that can hinder the recordings of interest. Also if the material of the provision tube 31 in contact with the skin is too flexible, it will deform, impeding the provision of the electrolytic gel 4 through said provision tube 31.

In another more preferred embodiment, the provision tube 31 is comprised of polypropylene, more preferably PP9074MED. Advantageously this provides a safe material, with good biocompatibility and mechanical properties.

In another more preferred embodiment, the provision tube 31 comprises at least two apertures 315 on its distal end, the distal end configured to face the skin of the user, wherein the at least two apertures 315 are laterally placed on the wall of the provision tube 31. More preferably, the at least two apertures 315 are laterally placed such that the opening 34 is extended through such at least two apertures 315 on the wall of the provision tube 31. Advantageously, the provision of the at least two apertures 315 on the distal end of the provision tube, prevents the hindering of administration of the predefined amount of electrolytic gel 4 between the at least one electrode 2 and the skin surface of the user, due to the Venturi effect. The Venturi effect is produced when the provision tube 31 is close to the skin of the subject, leading to a narrowing of the space wherein the predefined amount of electrolytic gel 4 can exit the dispensing unit 3, leading to a pressure drop that hinders the exit of the predefined amount of electrolytic gel 4 towards the skin of the user. In another more preferred embodiment, the provision tube 31 comprises the at least two apertures 315 equidistantly disposed from each other. In another more preferred embodiment, the provision tube 31 comprises exactly two apertures 315. Advantageously, any of these two more preferred embodiments provide a more uniform distribution of the predefined amount of electrolytic gel 4 between the at least one electrode 2 and the skin surface of the user.

It is noted that the length of the provision tube 31 allows the electrolytic gel 4 to save the distance between the dispensing unit 3 wherein the electrolytic gelt 4 is comprised and the space between the electrode 2 and the skin surface of the user. Thus the length of the provision tube 31 may vary depending on the thickness of the electrode 2, the thickness of the dispensing unit 2, the amount of electrolytic gel 2 to be disposed between the electrode 2 and the skin surface of the user and the amount of hair in the user's head. According to another more preferred embodiment, the provision tube 31 comprises a length between 5and 12 mm. In a particular more preferred embodiment, the provision tube 31 comprises a length between 6 and 11 mm, even more preferably between 7 and 10 mm or between 8 and 9 mm. Advantageously, this length allows the provision of the predefined amount of electrolytic gel 4 at a distance between the at least one electrode 2 and the skin surface of the user that favours its correct provision. In a particularly preferred embodiment, the length of the provision tube 31 is between 8,4 and 9 mm, more preferably 8,48 mm.

It is noted that Fig 12 comprises many features that are not essential and may be omitted or may be different in other embodiments of the present invention. For example, the dispensing unit 3 of Figs. 12A-12B comprises a particular opening 34 through which the electrolytic gel 4 is provided. However, in the embodiments the opening 34 can take other shape, be located in other place or the dispensing unit 3 may comprise more than one opening 34. Also, it is noted that the dispensing unit 3 comprises a pair of ears 33 sticking out that help in the actuation of the plunger 32. In other embodiments these structures 33 may take other shape, may be more than two or may not be present at all in the dispensing unit 3. Finally, it is noted that the provision tube 31 in Figs, 12A and 12B comprise a determined shape, and length, but in other embodiments the provision tube 31 can take other shape or be located in other place. Also the provision tube 31 of Figs 12A and 12B comprises two apertures 315 but in other embodiments the provision tube may not comprise these apertures 315, or may comprise more than two apertures 315, and they may be located in other place at the distal end of the provision tube 31.

it can be observed that the electrode 2 is significantly thinner than the dispensing unit 3, and that the dispensing unit is placed over the electrode 2 such that the electrode 2 is practically embedded within the dispensing unit 3. However, this may not be the case in other embodiments of this more preferred embodiment. The dispensing unit 3 and the plunger 32 may take many other different shapes. Also the dispensing unit of Figs. 3A-3C comprises a particular opening 34 through which the electrolytic gel 4 is provided. However, in the embodiments the opening 34 can take other shape, be located in other place or the dispensing unit 3 may comprise more than one opening 34. Finally, it is noted that the dispensing unit 3 comprises a pair of ears 33 sticking out that help in the actuation of the plunger 32. In other embodiments these structures 33 may take other shape, may be more than two or may not be present at all in the dispensing unit 3.

A second aspect of the invention refers to the use of the registering system according to any of the embodiments of the first aspect of the invention when the registering system is a EEG system, to register an EEG signal, preferably an evoked potential, more preferably an somatosensory evoked potential (SEP). Therefore, the second aspect of the invention might relate to the use of any of the registering systems of the first aspect of the invention to monitor any type of EEG signal, wherein the signal may be elicited by the natural activity of the patient or evoked externally through an stimulus or stimuli.

SEPs have multiple diagnostic and prognostic uses, such as predicting the outcome of an endovascular treatment before the endovascular treatment has been performed or the status of a certain neural pathway. For example, the SEPs measured before the endovascular treatment can provide an insight on the vascular situation of certain brain regions, for example the presence, severity and status of an ischemic stroke, and the prognosis of the endovascular treatment outcome, if applied. Furthermore, it can be also used to evaluate the progression of the treatment itself, such that measuring SEPs during the treatment, provides insights of the real time status of the vascular situation of the affected brain regions. This can be used to take decisions during the surgery. Another use of SEPs is the post-intervention monitoring and prediction of the recovery of such patients. Other uses include the evaluation of anoxic patients in the Intensive Care Unit, or cerebral function evaluation in emergency situations such as patients with decreased level of consciousness of unknown aetiology or with suspected status epilepticus or encephalopathy. Therefore, an EEG registering system according to any of the embodiments of the first aspect of the invention are specially desirable to register a somatosensory evoked potential (SEP) in complex, stressful, and/or time-stressed situations, even more when the situation is complex, stressful, and time-stressed.

According to a preferred embodiment of the second aspect of the invention, the registering system is used to register visual evoked potentials or auditory evoked potentials. Advantageously, the registering system would reduce the time per patient and increase the efficiency of the healthcare assistance.

In a preferred embodiment of the second aspect of the invention, the electroencephalography registering system is used to register the N20 SEP. Advantageously, the N20 SEP has been shown to be specially suitable to predict the outcome of an endovascular treatment before, during and after an endovascular treatment in patient suffering an ischemic stroke, including acute ischemic stroke.

A third aspect of the invention relates to a system to provide an electrolytic gel 4 between at least one electrode 2 and the skin surface of a user; wherein the electrolytic gel 4 is provided through an opening 34 defined within a provision tube 31, the provision tube 31 configured to close the gap between the dispensing unit 3 comprising the electrolytic gel 4 and the skin of the user. Advantageously, the provision tube 31 facilitates the provision of the electrolytic gel 4 between the at least one electrode 2 and the skin surface of the user.

In a more preferred embodiment, the provision tube 31 comprises a flexural modulus between 1100 and 1300MPa. Preferably the provision tube 31 comprises a flexural modulus between 1100 and 1200MPa, even more preferably a flexural modulus of essentially 1140 MPa. Advantageously, a provision tube 31 with a flexural modulus within these values provides a material firm enough to maintain its shape when provided onto the user's skin, but with enough flexibility to be gentle with the user's skin. This of much relevance in clinical applications, wherein the collaboration of the patient with the registration is essential to ensure an adequate recoding of the biosignals. If the user (patient) is not comfortable with the dispensing unit 1 because the material of the provision tube 31 in contact with the skin is too stiff, the user may sense a discomfort that can hinder the recordings of interest. Also if the material of the provision tube 31 in contact with the skin is too flexible, it will deform, impeding the provision of the electrolytic gel 4 through said provision tube 31.

In another more preferred embodiment, the provision tube 31 is comprised of polypropylene, more preferably PP9074MED. Advantageously this provides a safe material, with good biocompatibility and mechanical properties.

In another more preferred embodiment, the provision tube 31 comprises at least two apertures 315 on its distal end, the distal end configured to face the skin of the user, wherein the at least two apertures 315 are laterally placed on the wall of the provision tube 31. More preferably, the at least two apertures 315 are laterally placed such that the opening 34 is extended through such at least two apertures 315 on the wall of the provision tube 31. Advantageously, the provision of the at least two apertures 315 on the distal end of the provision tube, prevents the hindering of administration of the electrolytic gel 4 between the at least one electrode 2 and the skin surface of the user, due to the Venturi effect. The Venturi effect is produced when the provision tube 31 is close to the skin of the subject, leading to a narrowing of the space wherein the predefined amount of electrolytic gel 4 can exit the dispensing unit 3, leading to a pressure drop that hinders the exit of the predefined amount of electrolytic gel 4 towards the skin of the user. In another more preferred embodiment, the provision tube 31 comprises the at least two apertures 315 equidistantly disposed from each other. In another more preferred embodiment, the provision tube 31 comprises exactly two apertures 315. Advantageously, any of these two more preferred embodiments provide a more uniform distribution of the electrolytic gel 4 between the at least one electrode 2 and the skin surface of the user.

It is noted that the length of the provision tube 31 allows the electrolytic gel 4 to save the distance between the dispensing unit 3 wherein the electrolytic gelt 4 is comprised and the space between the electrode 2 and the skin surface of the user. Thus the length of the provision tube 31 may vary depending on the thickness of the electrode 2, the thickness of the dispensing unit 2, the amount of electrolytic gel 2 to be disposed between the electrode 2 and the skin surface of the user and the amount of hair in the user's head. According to another more preferred embodiment, the provision tube 31 comprises a length between 5and 12 mm. In a particular more preferred embodiment, the provision tube 31 comprises a length between 6 and 11 mm, even more preferably between 7 and 10 mm or between 8 and 9 mm.

Advantageously, this length allows the provision of the predefined amount of electrolytic gel 4 at a distance between the at least one electrode 2 and the skin surface of the user that favours its correct provision. In a particularly preferred embodiment, the length of the provision tube 31 is between 8,4 and 9 mm, more preferably 8,48 mm.

All of the above are fully within the scope of the present disclosure and are considered to form the basis for alternative embodiments in which one or more combinations of the above described features are applied, without limitation to the specific combination disclosed above.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this art. All such equivalents, modifications or adaptations fall within the scope of the present disclosure.

### EXAMPLES

### Example 1: Electrolytic gel performance

### Materials and methods

In this example, the experiment tested different electrolytic gels 4 in order to evaluate their impedance and the performance when used with a registering system according to the present invention.

In particular, 4 setups with different electrolytic gels were prepared:
- 2 regular electrolytic gels
   ∘ OneStep Clear Gel: an electrolytic gel without fat, colorants and easy to remove
   ∘ Neurgel: an elastic electrolytic gel that reduces the bridging between electrodes
- 2 electrolytic gels comprising an abrasive component
   ∘ OneStep EEG gel: an electrolytic gel with pumice
   ∘ OneStep AbrasivPlus gel: another an electrolytic gel with pumice and a higher abrasive effect

The impedance of the registering system with each of the electrolytic gels was computed through a commercially available system for the monitorization of neurophysiological signals which automatically computes the impedances of the electrodes.

### Results

The regular electrolytic gels has generally good impedance in all cases as shown in Table 1.

| Impedance (KΩ) | | | |
|---|---|---|---|
| | **C4'** | **Cz'** | **GND** |
| **OneStep Clear Gel** | 1.5 | 10-15 | 10-15 |
| **Neurgel** | 1.3 | 8 | 6-13 |
| **OneStep EEG gel** | 2.5 | 13 | 8 |
| **OneStep AbrasivPlus gel** | 1.1 | 9 | 7-11 |

Fig. 7 shows the evoked potential of the electrolytic gels comprising an abrasive component a) OneStep EEG gel and b) OneStep AbrasivPlus gel.

Fig. 8 shows the evoked potential of the regular electrolytic gels a) OneStep Clear Gel and b) Neurgel.

### Example 2: Electrolytic gel amount

### Materials and methods

In this example, the experiment tested different amounts of electrolytic gels 4 in order to evaluate their impedance and the performance when used with a registering system according to the present invention.

In particular, three setups with different amounts of electrolytic gel: 2 mL, 3 mL and 4 mL were prepared.

The electrodes in use had a surface area between the electrode and the skin surface of 1.727875 cm².

The impedance of the registering system with each of the electrolytic gels was computed through a commercially available system for the monitorization of neurophysiological signals which automatically computes the impedances of the electrodes.

### Results

The impedance of the different amounts of electrolytic gels is shown in Table 2.

| Impedance (KΩ) | | | | |
|---|---|---|---|---|
| | **C3'** | **C4'** | **Cz'** | **GND** |
| **2 mL** | 18 | 17 | 14,3 | 24 |
| **3 mL** | 17 | 16 | 12 | 3,4 |
| **4 mL** | 6,7 | 5 | 6,7 | 5,6 |

Fig. 9 shows a) the evoked potential and b) the detrended signal of the setup where 2mL of electrolytic gel was provided between the electrode and the user's skin. Fig. 10 shows a) the evoked potential and b) the detrended signal of the setup where 3mL of electrolytic gel was provided between the electrode and the user's skin. Fig. 11 shows a) the evoked potential and b) the detrended signal of the setup where 4mL of electrolytic gel was provided between the electrode and the user's skin.

Therefore it can be seen that it is required to have more than 3mL of electrolytic gel for an electrode contact surface of 1.727875 cm². In other words, it is required to provide more than 1,75 mL of electrolytic gel per cm² of surface of electrode in contact with the skin through said electrolytic gel to have a low impedance. Particularly, when 2.3 mL of electrolytic gel is provided per cm² of surface of electrode in contact with the skin through said electrolytic gel, the skin-electrode is optimal, as it is significantly lower than 10kΩ.

## Claims

1. A registering system comprising:
a. at least one electrode (2), and
b. at least one dispensing unit (3) configured to be placed over the at least one electrode (2) and provide a predefined amount of electrolytic gel (4) between the at least one electrode (2) and the skin surface of the user;
**characterised in that** the predefined amount of electrolytic gel (4) is of at least 1.75 ml per cm² of surface of electrode in contact with the skin through said electrolytic gel, more preferably at least 2.3 ml per cm² of surface of electrode in contact with the skin through said electrolytic gel.

2. The registering system according to claim 1, wherein the registering system is an electroencephalography registering system, further comprising a head mount (1) configured to attach to the head of a user, wherein the at least one electrode (2) is configured to be placed within the head mount (1) in a predefined location over the scalp of the user, and wherein the predefined amount of electrolytic gel (4) is provided between the at least one electrode (2) and the scalp surface.

3. The system according to any one of the previous claims, wherein the dispensing unit (3) is configured to provide the predefined amount of electrolytic gel (4) when a force is applied to a certain region of the dispensing unit (3).

4. The system according to claim 3, wherein the dispensing unit (3) comprises a plunger (32) or a flexible surface and the predefined amount of electrolytic gel (4) is provided when the force is applied to the plunger (32) or the flexible surface, respectively.

5. The system according to claim 4, wherein the dispensing unit (3) is configured to provide the predefined amount of electrolytic gel (4) when a rotational force is applied to the plunger (32).

6. The system according to any one of claims 1 or 2, wherein the dispensing unit (3) comprises a seal (35) and is configured to provide the predefined volume by gravity once the seal (35) has been defunctionalized.

7. The system according to claim 6, wherein the registering system further comprises a seal breaking device (5), configured to be attached to the dispensing unit (3) and to break the seal (35) by the attachment mechanism to the dispensing unit (3).

8. The system according to any one of the previous claims, wherein the at least one electrode (2) comprises an opening (25) and wherein the dispensing unit (3) is configured to provide the predefined amount of electrolytic gel though the opening (25).

9. The system according to any one of the previous claims, wherein the electrolytic gel (4) comprises an abrasive component, and wherein the dispensing unit (3) is further configured to prepare and clean the skin of the user through the provision of the electrolytic gel (4) comprising the abrasive component.

10. The system according to claim 9, wherein the abrasive component comprises pumice.

11. The system according to any one of the previous claims, wherein the at least one electrode (2) comprises any of the materials selected from the following list: gold, steel, silver, silver - silver chloride and sintered silver-silver chloride.

12. The system according to any one of the previous claims, wherein the system comprises more than one registering electrode (2a) and at least one ground electrode (2b).

13. The system according to claim 12 when dependent on claim 2, wherein the registering electrodes are located in evoked potential locations of the scalp, preferably in sensory evoked potential positions more preferably in the Cz', C3' and C4' respective locations of the head mount (1) according to the international 10-20 system, and wherein the ground electrode (2b) is located in a mastoid location of the head mount (1).

14. Use of the electroencephalography registering system according to any one of claims 1 to 13 when dependent on claim 2, to register an EEG signal, preferably an evoked potential, more preferably an somatosensory evoked potential (SEP).

15. Use of the electroencephalography registering system according to claim 14, wherein the EEG signal to be registered is the N20 SEP.
